# EUROPEAN PATENT SPECIFICATION

(11) **EP 2 911 530 B1**
(45) Date of publication and mention of the grant of the patent: **01.02.2017**
(21) Application number: 13779563.9
(22) Date of filing: 17.10.2013
(51) Int. Cl.: A61K 9/107, A61K 9/127, A61K 9/51, A23L 33/18

(54) **ENCAPSULATED BITTER PEPTIDES, METHODS OF ENCAPSULATING BITTER PEPTIDES, AND NUTRITIONAL COMPOSITIONS INCLUDING ENCAPSULATED BITTER PEPTIDES**
EINGEKAPSELTE BITTERE PEPTIDE, VERFAHREN ZUM EINKAPSELN BITTERE PEPTIDE UND NAHRUNGSZUSAMMENSETZUNGEN MIT VERKAPSELTEN BITTEREN PEPTIDEN
PEPTIDES AMERS ENCAPSULÉS, PROCÉDÉS D'ENCAPSULATION DE PEPTIDES AMERS, ET COMPOSITIONS NUTRITIONNELLES COMPRENANT DES PEPTIDES AMERS ENCAPSULÉS

(30) Priority: 25.10.2012 US 201261718523 P; 19.06.2013 US 201361836754 P
(43) Date of publication of application: 02.09.2015
(73) Proprietor: Nestec S.A., 1800 Vevey (CH)
(72) Inventor: SOUSSAN, Elodie, CH-3012 Bern (CH); MAYNARD, Françoise, 1093 La Conversion (CH)
(74) Representative: Stephen, Paula-Marie
(86) International application number: PCT/EP2013/071708
(87) International publication number: WO 2014/063985

(56) References cited:
- EP-A1- 1 897 451
- WO-A1-03/020319
- WO-A1-2010/039036
- WO-A2-2008/130236
- JP-A- H09 162
- US-A- 3 493 385
- US-A1- 2009 053 290
- DATABASE WPI Week 199018 Thomson Scientific, London, GB; AN 1990-135523 XP002720004, & JP H02 83031 A (LION CORP) 23 March 1990 (1990-03-23)

## Description

### BACKGROUND

The present disclosure relates generally to encapsulated bitter peptides, methods of encapsulating bitter peptides, and nutritional compositions including encapsulated bitter peptides. More specifically, the present disclosure is directed to bitter peptides encapsulated in a lipid matrix, wherein said lipid matrix is an organogel.

There is a growing need in the market for nutritional compositions that treat aging- and/or illness-related conditions and provide hydrolyzed protein. Protein hydrolyzates are commercially used specialized nutrition products for addressing specific consumers benefits and/or needs, such as for critical patients, and for prevention and management of allergy. Many such specialized nutrition products include proteins that are pre-digested by hydrolysis with an enzyme. However, hydrolysis may form hydrophobic fragments of proteins that have a bitter taste. See, e.g., Leksrisompong, P.P., "Characterization of flavor of whey protein hydrolysates," J. Agric. Food Chem., 58(10):6318-6327 (2010). Nevertheless, peptides not only contribute to the nutritional profile of the composition but also help stabilize the emulsion in the composition.

Due to the hydrophobic fragments of proteins formed in protein hydrolysis, such nutritional compositions have an undesirable taste from hydrolyzed protein that renders the composition unappealing for oral consumption. The desired biological result is not achieved when the consumer refuses to ingest the nutritional composition due to poor organoleptic properties of the composition. Thus, it would be beneficial to provide nutritional compositions having both hydrolyzed protein and tolerable physical and organoleptic properties, without impacting the emulsion stability and without compromising the bioavailability of the peptides.

The encapsulation of bitter peptides for taste-masking purposes is known from WO2010/039036, WO2008/130236, EP1897451 and JPH0283031.

### SUMMARY

The present disclosure provides encapsulated bitter peptides, methods of selectively encapsulating bitter peptides, and nutritional compositions including encapsulated bitter peptides. In an embodiment, a nutritional composition is provided and includes bitter peptides encapsulated in a structure, wherein said structure is anorganogel.

In an embodiment, the structure is an organogel comprising an oil and a gelator, for example distilled monoglycerides.

In another embodiment, a method of encapsulating bitter peptides is provided. The method includes the steps of forming an emulsion comprising a water phase containing protein hydrolysate and an oil phase comprising at least one of an oil or a melted fat; and adding a gelator to the emulsion to form an organogel after the bitter peptides from the protein hydrolysate migrate selectively into oil droplets in the oil phase, the addition of the gelator entrapping the bitter peptides in the organogel.

In an embodiment, the protein hydrolysate is a dairy based protein hydrolysate. In an embodiment, the dairy based protein hydrolysate protein is a whey protein hydrolysate. In another embodiment the protein hydrolysate is a vegetable based protein hydrolysate, such as pea and/or soy protein hydrolysate.

In an embodiment, the method includes hydrolysis of protein to form a solution comprising the protein hydrolysate in a water phase, the bitter peptides being selectively entrapped in the organogel in line such that the emulsion is formed after the hydrolysis directly in the solution comprising the protein hydrolysate.

In an embodiment, the hydrolysis forms non-bitter peptides, and at least a portion of the non-bitter peptides are not entrapped in the oil phase.

In an embodiment, the water phase contains about 5% to about 50% of the protein hydrolysate by weight, preferably about 5% to about 25%, such as about 5% to about 15% of the protein hydrolysate by weight.

In another embodiment, bitter peptides encapsulated in a structure, wherein said structure is an organogel.

In another embodiment, a method of providing nutrition is provided. The method includes administering a nutritional composition comprising bitter peptides from protein hydrolysate, the bitter peptides encapsulated in a structure, wherein said structure is an organogel.

An advantage of the present disclosure is to provide encapsulated bitter peptides.

Another advantage of the present disclosure is to provide improved nutritional compositions.

Still another advantage of the present disclosure is to provide nutritional compositions that have acceptable organoleptic properties.

Yet another advantage of the present disclosure is to hide bitter peptides to taste receptors by encapsulating the peptides.

Still another advantage of embodiments of the present disclosure is to encapsulate bitter peptides in line, such that hydrophobic interactions encapsulate the bitter peptides directly in the water phase after inactivation of the enzyme, or near line.

Yet another advantage of the present disclosure is to selectively encapsulate bitter peptides such that at least a portion of the peptides that assist emulsion stability are not encapsulated.

Additional features and advantages are described herein, and will be apparent from the following Detailed Description and the figures.

### BRIEF DESCRIPTION OF THE FIGURES

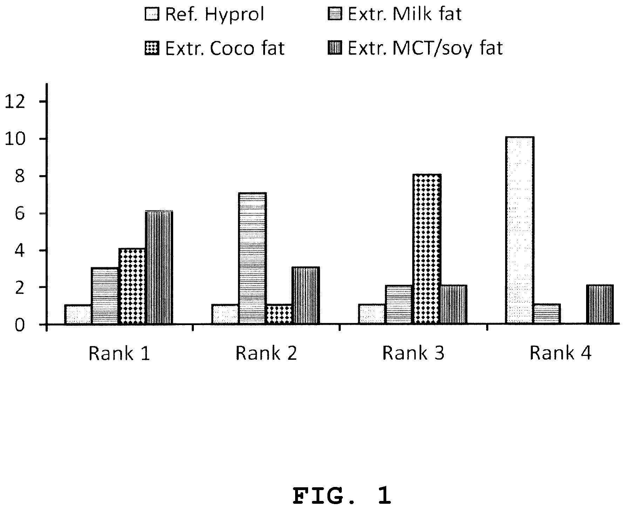

FIG. 1 is a graph showing results from tastings of encapsulated bitter peptides in accordance with embodiments of the present disclosure.

### DETAILED DESCRIPTION

All dosage ranges contained within this application are intended to include all numbers, whole or fractions, contained within said range.

As used in this disclosure and the appended claims, the singular forms "a," "an" and "the" include plural referents unless the context clearly dictates otherwise. Thus, for example, reference to "a polypeptide" includes a mixture of two or more polypeptides, and the like.

As used herein, "about" is understood to refer to numbers in a range of numerals. Moreover, all numerical ranges herein should be understood to include all integer, whole or fractions, within the range.

The present disclosure is related to bitter peptides encapsulated in a hydrophobic matrix. As used herein, "bitter peptides" are any hydrophobic peptide formed by hydrolysis of protein. In some embodiments, the bitter peptides include peptides that bind to the human bitter receptors (for examples T2Rs). The bitter peptides can be formed by hydrolysis of a dietary protein. Pre-hydrolysed proteins are generally more easily digested and absorbed by the gastro-intestinal tract, and have reduced allergenic potential.

The hydrolysate of any suitable dietary protein may be used, for example animal proteins, such as milk protein, meat protein and egg protein; or vegetable proteins, such as soy protein, wheat protein, rice protein, pea protein, corn protein, canola protein, oat protein, potato protein, peanut protein, and any proteins derived from beans, buckwheat or lentils. Milk proteins, such as casein and whey, and soy proteins may be preferred for some applications. If the protein is a milk protein or a milk protein fraction, the protein may be, for example, sweet whey, acid whey, α-lactalbumin, β-lactoglobulin, bovine serum albumin, acid casein, caseinates, α-casein, β-casein and/or γ-casein. Of course, hydrolysate from a combination of different proteins may be used.

Protein hydrolysates can be obtained as a commercial raw material powder or can be produced by hydrolysis of protein, e.g. enzymatic hydrolysis of protein. For example, according to an embodiment, protein hydrolysates may be produced by hydrolysis of whey protein in water using, proteolitic enzymes. Processes and enzymes for the preparation of protein hydrolysates for food applications are well-known in the art. One example of a process for preparation of protein hydrolysates is described in US5,039,532.

The peptides may be produced by hydrolyzing protein as desired and as known in the art. For example, protein hydrolysate, e.g. a whey protein hydrolysate, may be prepared by enzymatically hydrolysing the protein in one or more steps. In some embodiments, the protein can be obtained already hydrolyzed.

Applicants surprisingly found that encapsulating bitter peptides masked the bitterness relative to un-encapsulated bitter peptides from protein hydrolysates. Without wishing to be bound to any theory, it is believed that the encapsulated bitter peptides reduce undesirable taste of a composition in which they are included because the hydrophobic matrix in which they are encapsulated blocks the bitter peptides from taste receptors.

In some embodiments, at least a portion of the bitter peptides from the protein hydrolysate can be encapsulated in an organogel. An organogel is a substantially dilute system which exhibits no flow when in the steady-state and is a non-crystalline, non-glassy solid material. These solid materials are composed of a liquid organic phase (e.g vegetable oil) entrapped in and/or physically attached to a three-dimensional network. For example, an organogel can comprise macromolecules interconnected by either strong chemical bonds, such as the bonds of cross-linked polymers, or weaker bonds, such as non-covalent interactions. These systems can be based on self-assembly of structurant molecules called gelators (e.g. monoglyceride). Immobilization of the liquid component within the networked structure of the solid component has been attributed to the interfacial tension between the solid and liquid components.

To produce an organogel, an emulsion can be formed. In some embodiments, the emulsion is formed after protein hydrolysis directly in the water phase after inactivation of the enzyme. The emulsion can comprise a water phase containing the protein hydrolysate and also comprise an oil phase containing oil and/or melted fat. For example, protein hydrolysate, such as whey protein hydrolysate, can be dispersed in vegetable oil, such as sunflower oil. In an embodiment, the water phase can comprise about 5% to about 50% of the protein hydrolysate by weight, preferably about 5% to about 25%, such as about 5% to about 15% of the protein hydrolysate by weight.

The bitter peptides can initially be located in the water phase but then can migrate inside the oil droplets by hydrophobic interactions. For example, stirring, such as for about 30 minutes, can assist the bitter peptides in migrating inside the oil droplets. After the emulsion is formed, gelator molecules, such as, for example, phytosterols, phospholipids, monoglycerides and combinations thereof, can be added to the emulsion to gel the oil droplets in which the bitter peptides will be entrapped. Examples of suitable gelators include low molecular weight gelators (LMWGs), such as phospholipids, lecithin, monoglyceride, amphiphilic peptides, sorbitan monostearate, mono-, di- and/or triacyglycerols, fatty acids, fatty alcohol, wax and/or phytosterol. For example, a gelator can be added to the emulsion to gel the oil droplets. In a preferred embodiment, the organogel is formed using only food grade materials and without using any organic solvents. In a particular embodiment the gelator may be distilled monoglycerides.

The organogel can be used to produce a nutritional composition. In some embodiments, the oil and/or melted fat are standard ingredients in a nutritional composition in which the organogel that encapsulates the bitter peptides is used. For example, the formulation of the nutritional composition may not need to be altered to include the organogel. In some embodiments, at least a portion of the peptides from the protein hydrolysate that are not bitter and/or not hydrophobic are not encapsulated by the organogel. In some embodiments, the solid lipid nanoparticles are stabilized by a surfactant.

The present disclosure provides nutritional compositions comprising encapsulated bitter peptides produced using any method disclosed herein. The present disclosure also provides a method of administering bitter peptides to an individual comprising the steps of administering to the individual a nutritional composition comprising the bitter peptides in encapsulated form. The present disclosure also provides a method of treating or preventing a condition in an individual in need thereof comprising the steps of administering to the individual a nutritional composition comprising an effective amount of encapsulated bitter peptides.

For example, in an embodiment, a method of providing nutrition to a person comprises administering to the person a nutritional formulation comprising bitter peptides from protein hydrolysate. The bitter peptides are encapsulated in a structure selected from the group consisting of organogels, liposomes, solid lipid nanoparticles and combinations thereof.

The inventive encapsulation system advantageously liberates the encapsulated peptides during digestion so that the bitter taste of the peptides is masked on consumption/administration of the nutritional composition, whilst maintaining the biological properties of the hydrolysate, such that the hydrophobic peptides are still bioavailable.

Nutritional compositions comprising the encapsulated bitter peptides may be pharmaceutical formulations, nutritional formulations, dietary supplements, functional foods, beverage products and combinations thereof. The nutritional composition can be for long-term administration (continuous administrations for more than 6 weeks and/or short-term administration (continuous administrations for less than 6 weeks).

In an embodiment, the nutritional compositions are in a form selected from the group consisting of tablets, capsules, liquids, chewables, soft gels, sachets, powders, syrups, liquid suspensions, emulsions, solutions, or combinations thereof. In an embodiment, the nutritional compositions are oral nutritional supplements.

The nutritional compositions may also be a source of complete nutrition. Complete nutrition provides types and levels of macronutrients (protein, fats and carbohydrates) and micronutrients to be sufficient to be a sole source of nutrition for the animal to which it is being administered. Patients can receive 100% of their nutritional requirements from such complete nutritional compositions. Alternatively, the nutritional compositions may be a source of incomplete nutrition. Incomplete nutrition does not provide levels of macronutrients (protein, fats and carbohydrates) or micronutrients to be sufficient to be a sole source of nutrition for the animal to which it is being administered. Partial or incomplete nutritional compositions can be used as a nutritional supplement. According to another embodiment the nutritional composition can be an infant formula.

"Nutritional products," or "nutritional compositions," as used herein, are understood to include any number of optional additional ingredients, including conventional food additives (synthetic or natural), for example one or more acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamins. The optional ingredients can be added in any suitable amount. The nutritional products or compositions may be a source of complete nutrition or may be a source of incomplete nutrition.

The nutritional compositions can include any number of optional additional ingredients, including conventional food additives (synthetic or natural), for example one or more acidulants, additional thickeners, buffers or agents for pH adjustment, chelating agents, colorants, emulsifiers, excipients, flavor agents, osmotic agents, pharmaceutically acceptable carriers, preservatives, stabilizers, sugar, sweeteners, texturizers, and/or vitamins. For example, the nutritional compositions may contain emulsifiers and stabilizers such as soy lecithin, citric acid esters of mono- and di-glycerides, and the like. The optional ingredients can be added in any suitable amount.

Nutritional compositions of the present disclosure may contain a carbohydrate source. Any carbohydrate source conventionally found in nutritional compositions such as lactose, saccharose, maltodextrin, starch and mixtures thereof may be used although the preferred source of carbohydrates is lactose. In an embodiment, the carbohydrate source contributes between 35% and 60% of the total energy of the nutritional compositions.

Nutritional compositions of the present disclosure may also contain a source of lipids in addition to the lipids from the encapsulated bitter peptides. The lipid source may be any lipid or fat which is suitable for use in nutritional compositions. Sources of fat include, but are not limited to, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between about 10% and about 10% of the total energy of the nutritional compositions.

In an embodiment, the nutritional compositions further include a source of ω-3 fatty acids and/or a source of ω-6 fatty acids. The source of ω-3 fatty acids may be selected from the group consisting of fish oil, krill, plant sources containing ω-3 fatty acids, flaxseed, walnut, algae, or combinations thereof. The ω-3 fatty acids may be selected from the group consisting of α-linolenic acid ("ALA"), docosahexaenoic acid ("DHA"), eicosapentaenoic acid ("EPA"), or combinations thereof. The source of ω-6 fatty acids may be selected from the group consisting of vegetable oils (e.g. sunflower, sassflower, soybean, corn, sesame, cottonseed, grapeseed, palm, primerose, borage and walnut), nuts (e.g. walnuts, almonds and cashews), and seeds (e.g. flax, hemp, sunflower, sesame, pine nuts, black current and pumpkin).

In an embodiment, the nutritional compositions can include at least one nucleotide selected from the group consisting of a subunit of deoxyribonucleic acid ("DNA"), a subunit of ribonucleic acid ("RNA"), polymeric forms of DNA and RNA, yeast RNA, or combinations thereof. In an embodiment, the at least one nucleotide is an exogenous nucleotide.

In an embodiment, the nutritional compositions further include a phytonutrient selected from the group consisting of flavanoids, allied phenolic compounds, polyphenolic compounds, terpenoids, alkaloids, sulphur-containing compounds, or combinations thereof. The phytonutrient may be selected from the group consisting of carotenoids, plant sterols, quercetin, curcumin, limonin, or combinations thereof.

The nutritional compositions can further include a source of protein in addition to the encapsulated bitter peptides and/or the remainder of the hydrolysate. Any suitable dietary protein may be used, for example animal proteins, such as dairy protein, meat protein and egg protein; or vegetable proteins, such as soy protein, wheat protein, rice protein, pea protein, corn protein, canola protein, oat protein, potato protein, peanut protein, and any proteins derived from beans, buckwheat or lentils. The dairy based proteins may be casein, caseinates, casein hydrolysate, whey, whey hydrolysates, whey concentrates, whey isolates, milk protein concentrate, milk protein isolate, or combinations thereof. Dairy proteins, such as casein and whey, and/or soy proteins may be preferred for some applications. In an embodiment, the protein source contributes between 15% and 35% of the total energy of the nutritional compositions.

The nutritional compositions can further include a probiotic. Probiotics are food-grade microorganisms (alive, including semi-viable or weakened, and/or non-replicating), metabolites, microbial cell preparations or components of microbial cells that could confer health benefits on the host when administered in adequate amounts, more specifically, that beneficially affect a host by improving its intestinal microbial balance, leading to effects on the health or well-being of the host. See Salminen S., et al., "Probiotics: how should they be defined?," Trends Food Sci. Technol., 10, 107-10 (1999). In general, it is believed that these micro-organisms inhibit or influence the growth and/or metabolism of pathogenic bacteria in the intestinal tract. The probiotics may also activate the immune function of the host.

Non-limiting examples of probiotics include *Aerococcus, Aspergillus, Bacteroides, Bifidobacterium, Candida, Clostridium, Debaromyces, Enterococcus, Fusobacterium, Lactobacillus, Lactococcus, Leuconostoc, Melissococcus, Micrococcus, Mucor, Oenococcus, Pediococcus, Penicillium, Peptostrepococcus, Pichia, Propionibacterium, Pseudocatenulatum, Rhizopus, Saccharomyces, Staphylococcus, Streptococcus, Torulopsis, Weissella,* or combinations thereof.

In an embodiment, the nutritional compositions further include an amino acid selected from the group consisting of alanine, arginine, asparagine, aspartate, cysteine, glutamate, glutamine, glycine, histidine, hydroxyproline, hydroxyserine, hydroxytyrosine, hydroxylysine, isoleucine, leucine, lysine, methionine, phenylalanine, proline, serine, taurine, threonine, tryptophan, tyrosine, valine, or combinations thereof.

In an embodiment, the nutritional compositions further include an antioxidant. Non-limiting examples of antioxidants include astaxanthin, carotenoids, coenzyme Q10 ("CoQ10"), flavonoids, glutathione, Goji (wolfberry), hesperidin, lactowolfberry, lignan, lutein, lycopene, polyphenols, selenium, vitamin A, vitamin C, vitamin E, zeaxanthin, and combinations thereof.

In an embodiment, the nutritional compositions further include a vitamin selected from the group consisting of vitamin A, Vitamin B1 (thiamine), Vitamin B2 (riboflavin), Vitamin B3 (niacin or niacinamide), Vitamin B5 (pantothenic acid), Vitamin B6 (pyridoxine, pyridoxal, or pyridoxamine, or pyridoxine hydrochloride), Vitamin B7 (biotin), Vitamin B9 (folic acid), and Vitamin B12 (various cobalamins; commonly cyanocobalamin in vitamin supplements), vitamin C, vitamin D, vitamin E, vitamin K, K1 and K2 (i.e., MK-4, MK-7), folic acid, biotin, or combinations thereof.

In an embodiment, the nutritional compositions further include a mineral selected from the group consisting of boron, calcium, chromium, copper, iodine, iron, magnesium, manganese, molybdenum, nickel, phosphorus, potassium, selenium, silicon, tin, vanadium, zinc, or combinations thereof. Minerals may be added in salt form. The presence and amounts of specific minerals and other vitamins will vary depending on the intended population.

In an embodiment, the nutritional composition includes branched chain fatty acids that are present in the nutritional composition in an amount from about 6.25 mg to about 12.5 mg/100 g nutritional composition and assuming the nutritional composition includes 1,600 grams and is a complete feeding for a day for an adult. Alternatively, the nutritional compositions may be provided in an amount to provide from about 100 mg to about 1,500 mg of branched chain fatty acids per day. Alternatively, the nutritional compositions may include from about 0.5% to about 5% branched chain fatty acids by weight of total fatty acids.

In an embodiment, the nutritional compositions also include a prebiotic. A prebiotic is a food substance that selectively promotes the growth of beneficial bacteria or inhibits the growth or mucosal adhesion of pathogenic bacteria in the intestines. They are not inactivated in the stomach and/or upper intestine or absorbed in the gastrointestinal tract of the person ingesting them, but they are fermented by the gastrointestinal microflora and/or by probiotics. Prebiotics are, for example, defined by Glenn Gibson et al., "Dietary Modulation of the Human Colonic Microbiota: Introducing the Concept of Prebiotics," J. Nutr., 125: 1401-1412 (1995).

Non-limiting examples of prebiotics include acacia gum, alpha glucan, arabinogalactans, beta glucan, dextrans, fructooligosaccharides, fucosyllactose, galactooligosaccharides, galactomannans, gentiooligosaccharides, glucooligosaccharides, guar gum, inulin, isomaltooligosaccharides, lactoneotetraose, lactosucrose, lactulose, levan, maltodextrins, milk oligosaccharides, partially hydrolyzed guar gum, pecticoligosaccharides, resistant starches, retrograded starch, sialooligosaccharides, sialyllactose, soyoligosaccharides, sugar alcohols, xylooligosaccharides, or their hydrolysates, or combinations thereof.

In some embodiments, the nutritional composition can be a synbiotic that contains both a prebiotic and a probiotic that work together to improve the microflora of the intestine.

By way of example and not limitation, the following Examples are illustrative of embodiments of the present disclosure.

### EXAMPLES

The following examples present scientific data developing and supporting the concept of encapsulating bitter peptides from protein hydrolysate.

### Example 1

To ensure that bitter peptides migrate into the oil phase of organogels and solid lipid nanoparticles, an emulsion was created containing oil or melted fat and a water phase in which 10% of Hyprol™ 3315 was dissolved. After 30 minutes of stirring, the water phase and the oil phase were separated by decantation. The extracted water phase was then tasted by a trained panel. It is important to note that the extraction step is used for evaluation and would not be used in most embodiments of the encapsulated bitter peptides that are used in nutritional compositions.

Samples were prepared with milk fat, cocoa fat or a mixture of medium chain triglycerides (MCT) and soy lecithin. Each of the oil/fats were added at an amount of 4% by weight to the 10% Hyprol™ 3315 solution before extraction. Thirteen trained panelists ranked the samples from the least bitter (rank 1) to the most bitter (rank 4). As shown in Figure 1, the cocoa fat sample was slightly less bitter than the reference, and the hydrolysates extracted with milk fat and the mixture of MCT/soy lecithin were perceived as significantly less bitter than the reference. A Friedman test was performed on the results (global risk 5%) and concluded that the perceived decrease in bitterness relative to the control for the milk fat and MCT/soy fat extracts was statistically significant.

### Example 2

To further examine encapsulation of bitter peptides in an organogel, organogels were formed by dispersing Hyprol™ 3315 in sunflower oil and then adding a monoglyceride gelator (Dimodan™). After gellation, the bitterness of reconstituted solutions of 10% Hyprol™ 3315 were significantly reduced, meaning that the hydrophobic bitter peptides were encapsulated in organogel and were not released from organogel in water.

## Claims

1. A nutritional composition comprising bitter peptides encapsulated in a structure, wherein said structure is an organogel comprising an oil or melted fat and a gelator selected from the list consisting of phospholipids, lecithin, monoglyceride, amphiphilic peptides, sorbitan monostearate, mono-, di- and/or triacyglycerols, fatty acids, fatty alcohol, wax, phytosterol, and/or combinations thereof.

2. A method of encapsulating bitter peptides, the method comprising the steps of:
forming an emulsion comprising a water phase containing protein hydrolysate and an oil phase comprising at least one of an oil or a melted fat; and
adding a gelator to the emulsion to form an organogel after the bitter peptides from the protein hydrolysate migrate into oil droplets in the oil phase, the addition of the gelator entrapping the bitter peptides in the organogel.

3. The method of Claim 2 comprising hydrolysis of a protein to form a solution comprising the protein hydrolysate, the bitter peptides being entrapped in the organogel in line such that the emulsion is formed after the hydrolysis directly in the solution comprising the protein hydrolysate.

4. The method of any one of Claims 2 or 3, wherein the protein hydrolysate is a dairy protein hydrolysate.

5. The method of any one of Claims 2 or 3, wherein the hydrolysis forms non-bitter peptides, and at least a portion of the non-bitter peptides are not entrapped in the oil phase.

6. Bitter peptides encapsulated in a structure, wherein said structure is an organogel.

7. A method of providing nutrition to a person, comprising administering to the person a nutritional composition comprising bitter peptides from protein hydrolysate, the bitter peptides encapsulated in a structure, wherein said structure is an organogel.

## Patentansprüche

1. Nahrungsmittelzusammensetzung, umfassend bittere Peptide, die in einer Struktur eingekapselt sind, wobei die Struktur ein Organogel ist, das ein Öl oder geschmolzenes Fett und einen Gelator umfasst, der ausgewählt ist aus der Liste bestehend aus Phospholipiden, Lecithin, Monoglycerid, amphiphilen Peptiden, Sorbitanmonostearat, Mono-, Di- und/oder Triacyglycerinen, Fettsäuren, Fettalkohol, Wachs, Phytosterol und/oder Kombinationen davon.

2. Verfahren zum Einkapseln von bitteren Peptiden, wobei das Verfahren die folgenden Schritte umfasst:
Bilden einer Emulsion, die eine Wasserphase umfasst, die ein Proteinhydrolysat und eine Ölphase enthält, die mindestens eines von einem Öl oder einem geschmolzenen Fett umfasst; und
Zugeben eines Gelators zu der Emulsion, um ein Organogel zu bilden, nachdem die bitteren Peptide aus dem Proteinhydrolysat in Öltröpfchen in die Ölphase migrieren, wobei die Zugabe des Gelators die bitteren Peptide in dem Organogel einfängt.

3. Verfahren nach Anspruch 2, umfassend die Hydrolyse eines Proteins, um eine Lösung zu bilden, die das Proteinhydrolysat umfasst, wobei die bitteren Peptide in dem Organogel derart in Reihe eingefangen werden, dass die Emulsion nach der Hydrolyse direkt in der Lösung gebildet wird, die das Proteinhydrolysat umfasst.

4. Verfahren nach einem der Ansprüche 2 oder 3, wobei das Proteinhydrolysat ein Milchproteinhydrolysat ist.

5. Verfahren nach einem der Ansprüche 2 oder 3, wobei die Hydrolyse nicht bittere Peptide bildet, und wobei mindestens ein Anteil der nicht bitteren Peptide nicht in der Ölphase eingefangen ist.

6. Bittere Peptide, die in einer Struktur eingekapselt sind, wobei die Struktur ein Organogel ist.

7. Verfahren zum Bereitstellen von Nahrung für eine Person, umfassend das Verabreichen einer Nahrungsmittelzusammensetzung, die bittere Peptide aus Proteinhydrolysat umfasst, an die Person, wobei die bitteren Peptide in einer Struktur eingekapselt sind, wobei die Struktur ein Organogel ist.

## Revendications

1. Composition nutritionnelle comprenant des peptides amers encapsulés dans une structure, dans laquelle ladite structure est un organogel comprenant une huile ou une matière grasse fondue et un gélifiant choisi parmi la liste constituée de phospholipides, lécithine, monoglycéride, peptides amphiphiles, monostéarate de sorbitan, mono-, di- et/ou tri-acyglycérols, acides gras, alcool gras, cire, phytostérol, et/ou leurs combinaisons.

2. Procédé d'encapsulation de peptides amers, le procédé comprenant les étapes consistant à :
former une émulsion comprenant une phase aqueuse contenant un hydrolysat de protéines et une phase huileuse comprenant au moins l'une parmi une huile ou une matière grasse fondue ; et
ajouter un gélifiant à l'émulsion pour former un organogel après que les peptides amers provenant de l'hydrolysat de protéines migrent dans des gouttelettes d'huile dans la phase huileuse, l'addition du gélifiant piégeant les peptides amers dans l'organogel.

3. Procédé selon la revendication 2, comprenant l'hydrolyse d'une protéine pour former une solution comprenant l'hydrolysat de protéines, les peptides amers étant piégés dans l'organogel en ligne de telle sorte que l'émulsion est formée après l'hydrolyse directement dans la solution comprenant l'hydrolysat de protéines.

4. Procédé selon l'une quelconque des revendications 2 ou 3, dans lequel l'hydrolysat de protéines est un hydrolysat de protéines laitières.

5. Procédé selon l'une quelconque des revendications 2 ou 3, dans lequel l'hydrolyse forme des peptides non amers, et au moins une partie des peptides non amers n'est pas piégée dans la phase huileuse.

6. Peptides amers encapsulés dans une structure, dans lesquels ladite structure est un organogel.

7. Procédé de fourniture d'une nutrition à une personne, comprenant l'administration à la personne d'une composition nutritionnelle comprenant des peptides amers provenant d'un hydrolysat de protéines, les peptides amers étant encapsulés dans une structure, dans lequel ladite structure est un organogel.
